# EUROPÄISCHE PATENTANMELDUNG

(11) **EP 2 133 085 A1**
(43) Veröffentlichungstag der Anmeldung: **16.12.2009**
(21) Anmeldenummer: 08010494.6
(22) Anmeldetag: 10.06.2008
(51) Int. Cl.: A61K 35/12, A61K 8/98, A61P 17/02

(54) **Verfahren zur Gewinnung eines Protein-Hydrolysats ohne Antigene aus den Füßen von immunologisch unreifem Geflügel**

(71) Anmelder: Eikenberg, Klaus, 59427 Unna (DE)
(72) Erfinder: Eikenberg, Klaus, 59427 Unna (DE)

(57) **Zusammenfassung**

Verfahren zur Gewinnung eines Protein-Hydrolysats aus den Füßen von immunologisch unreifem Geflügel.

Das Protein, so erzeugt, ist ein Auszug aus Polypeptiden und Aminosäuren. Es eignet sich als Pulver, Salbe, Gel, Creme durch Auftragen auf die Hautoberfläche von Menschen oder Tieren zur kosmetischen Unterstützung von Hautirritationen, Linderung bei Muskelkater und Verspannungen sowie Muskel- oder Nervengewebeschäden und beschleunigter Wundheilung sowie narbigem Gewebe und als wässerige Lösung als Injektion zur Zellregeneration.

Das Verfahren umfasst die Reinigung von Hühnerfüßen oder anderer tierischer Proteinquellen und Weiterbehandlung mit verdünnter Säure, vorzugsweise Essigsäure, Erhitzung und Trocknung zur Gewinnung eines Pulvers, Salbe oder Gels zur wiederholten Oberflächenbehandlung von Haut, Muskel- und Nervengewebe. Auch kann eine wässerige Lösung des Proteins unterspritzt werden.

## Beschreibung

(1) Hintergrund des Verfahrens zur Behandlung von Haut, Muskel- und Nervengewebe ist die Überlegung, dass die höchste Regenerationskraft zur Zellerneuerung mit einem Protein in einem möglichst frühen Stadium der Zellentwicklung zu erfolgen hat. Ähnlich einer Zelle im Embryonalstadium, die sich zu Muskeln, Nerven, Haut, Organen usw. ausbilden kann, müsste auch ein Protein diese Eigenschaften aufweisen, um so eine optimale Zellregeneration zu erzeugen. Das Ausgangsmaterial müsste billig und leicht verfügbar sein.
(2) Dieses Merkmal weisen frisch geschlachtete und immunologisch unreife Tiere und Geflügel auf, deren Antikörper noch nicht voll entwickelt sind. Das Hindernis bei Heilungsprozessen mit Proteinen ist die Antigenität. Nur Zellen, die noch nicht voll entwickelt sind, verfügen nicht über die Fähigkeit der Abstoßung fremder Proteine. Solch eine unreife Protein-Quelle ist in der Lage, Zellen mit der Information eines nahezu embryonalen Zustands zu induzieren, so dass diese körpereigenen Zellen, so angeregt, Zellregeneration beschleunigen oder neue Zellen produzieren.
(3) Auch im Erwachsenenstadium enthält der menschliche Körper noch eine gewisse Anzahl von Stammzellen, wie z.B. in der Leber, Haut, Gehirn, Knochenmark. Sie sind aber nur in sehr geringer Anzahl und schwer zu isolieren. Bislang fehlt eine Methode der Umprogrammierung dieser noch im menschlichen Körper verbliebenen Stammzellen, also einer stimulierenden Umwandlung von pluripotenten oder multipotenten Stammzellen in gewünschte Zelltypen.
(4) Das von immunologisch unreifen Hühnern hergestellte Extrakt besitzt die Eigenschaft des Informationstransfers auf geschädigte Zellstrukturen zur Zellerneuerung ähnlich dem Prozess der selbstheilenden Fingerschnittwunde und sorgt so für Regeneration von z.B. Hautirritationen, Muskelkater, Muskelverspannungen und narbigem Gewebe bei gleichzeitiger Zellregeneration und Beschleunigung der Wundheilung.

## Patentansprüche

1. Herstellungsprozess zur Gewinnung eines Extrakts als Pulver, Gel, Salbe oder Flüssigkeit zur kosmetischen Behandlung und Regeneration von Muskelverspannungen, Muskel- Nerven- und Hautverletzungen und Narben sowie Beschleunigung der Wundheilung der **dadurch gekennzeichnet ist, dass** man Körpermaterial von immunologisch unreifen geschlachteten Tieren entnimmt.

2. Der Prozess der Gewinnung von immunologisch unreifem Körpermaterial von Tieren ist **dadurch gekennzeichnet,** das man Geflügel nimmt, das nicht älter als 9 Wochen ist oder von anderen Proteinquellen immunologisch unreifer frisch getöteter Tiere.

3. Das tierische Körpermaterial ist **dadurch gekennzeichnet,** das es sich vorzugsweise um Geflügelfüße handelt, die von Hühnern und Hähnchen stammen, die nicht älter als 9 Wochen und immunologisch unreif sind.

4. Der Herstellungsprozess ist **dadurch gekennzeichnet, dass** die Geflügelfüße mit Wasser von Verschmutzungen gründlich gereinigt werden.

5. Der Herstellungsprozess ist **dadurch gekennzeichnet,** das die Geflügelfüße zerkleinert und gemahlen werden

6. Der Herstellungsprozess ist **dadurch gekennzeichnet, dass** eine schwache organische Säure angesetzt wird.

7. Die schwache organische Säure ist **dadurch gekennzeichnet, dass** es sich um eine 1 - 1,5 %ige Essigsäure-Lösung oder Milchsäure-Lösung handelt.

8. Der Herstellungsprozess ist **dadurch gekennzeichnet, dass** die zerkleinerten Geflügelfüße zur Entfernung von Blut, Serum und Fetten in die schwache organische Säure für 8 - 12 Stunden bei einer Zimmertemperatur von 21° - 32° Celsius bei gelegentlichem Umrühren gelegt werden.

9. Der Herstellungsprozess ist **dadurch gekennzeichnet, dass** die zerkleinerten Geflügelfüße mit Wasser gespült werden.

10. Der Herstellungsprozess ist **dadurch gekennzeichnet, dass** eine zweite organische Säure angesetzt wird.

11. Die organische Säure ist **dadurch gekennzeichnet, dass** sie als Wasser-Essigsäure wahlweise einen pH-Wert zwischen 3,6 und 6,5 aufweist.

12. Der Herstellungsprozess ist **dadurch gekennzeichnet, dass** die zerkleinerten Geflügelfüße in die organische Säure eingelegt werden und erhitzt werden.

13. Der Herstellungsprozess ist **dadurch gekennzeichnet, dass** die Erhitzung mit einer Temperatur von 54° - 60° Celsius für ca. 30 - 40 Minuten erfolgt, um so eine gelöste Proteinmischung zu erhalten.

14. Der Herstellungsprozess ist **dadurch gekennzeichnet, dass** die gelöste Proteinmischung in einer Zentrifuge entfettet wird.

15. Der Herstellungsprozess ist **dadurch gekennzeichnet, dass** die gelöste Proteinmischung gefiltert wird.

16. Der Herstellungsprozess ist **dadurch gekennzeichnet, dass** von der gelösten Proteinmischung noch vorhandene Fette abgeschöpft werden.

17. Der Herstellungsprozess ist **dadurch gekennzeichnet, dass** die gelöste Proteinmischung durch Erhitzung auf 37° - 49° Celsius verflüssigt wird.

18. Der Herstellungsprozess ist **dadurch gekennzeichnet, dass** man durch die erneute Erhitzung eine verflüssigte Lösung von Aminosäuren und Peptiden erhält, die einen 4-9 %igen fein strukturierten Festanteil enthält.

19. Der Herstellungsprozess einer gelartigen Lösung ist **dadurch gekennzeichnet, dass** die verflüssigte Lösung bei Raumtemperatur getrocknet wird.

20. Der Herstellungsprozess eines Pulvers ist **dadurch gekennzeichnet, dass** die Lösung in flüssigem Zustand bei einer Temperatur von 37° - 49° Celsius durch einen Sprühtrockner mit einer Arbeitstemperatur von 121° Celsius geführt wird.

21. Der Herstellungsprozess des Pulvers ist **dadurch gekennzeichnet,** das nach dem Sprühtrocknungsprozess ein ohne Antigene enthaltendes Protein-Hydrolysat-Pulver entsteht, das in einem feuchtigkeitsunempfindlichen luftdichten Container aufbewahrt wird.

22. Das keine Antigene enthaltende Protein-Hydrolysat-Pulver ist **dadurch gekennzeichnet, dass** es folgende Inhaltsstoffe aufweist:
- Wasser pH-Wert 4,6
- Asche
- Aminosäureverteilung:
- Asparaginsäure
- Glutaminsäure
- Histidin
- Lysin
- Argenine
- Threonin
- Serin
- Tyrosin
- Glycin
- Zystin
- Prolin
- Alanin
- Valin
- Methionin
- Leucin
- Isoleucin
- Phenylalanin

23. Die Inhaltsstoffe des Protein-Hydrolysats sind **dadurch gekennzeichnet, dass** sie mit jedem anderen üblicherweise verwendeten Inhaltsstoff angewendet werden können, wie zum Beispiel mit Kolloidalem Silber.

24. Die Verbindungen des Protein-Hydrolysats sind **dadurch gekennzeichnet, dass** sie in Form einer Emulsion wie die Makro-, Mikro-, oder Nanokapseln, Injektionslösung, Dispersion oder eingekapselt in Träger die Makro-, Mikro-, oder Nanokapseln, in Liposomen oder Chylomikronen, oder eingeschlossen in Makro-, Mikro-, oder Nanoteilchen oder in Mikroschwämme oder absorbiert auf pulverförmigen organischen Polymeren, Bentonit, Talk, und anderen mineralischen Trägem verwendet werden.

25. Die Verbindungen des Protein-Hydrolysats sind **dadurch gekennzeichnet, dass** sie in jeder Form verwendet werden: Pflaster, Gesichtsmasken, Emulsionen Öl/Wasser und Wasser/Öl, Milch, Lotionen, Salben, gelierende und viskose, spannungsaktive und emulgierende Polymere, Seifen, Gele, Puder, Sticks und Stifte, Sprays, Körperöle, Shampoos und Pommaden.
